# EUROPEAN PATENT APPLICATION

(11) **EP 4 666 933 A1**
(43) Date of publication of application: **24.12.2025**
(21) Application number: 24208021.6
(22) Date of filing: 22.10.2024
(51) Int. Cl.: A61B 3/16, G02B 1/04, G02C 7/04, G02C 11/00

(54) **INTRAOCULAR PRESSURE SENSING ELEMENT AND INTRAOCULAR PRESSURE SENSING METHOD**

(30) Priority: 20.06.2024 TW 113122802
(71) Applicant: Azurewave Technologies, Inc., 231 New Taipei City (TW)
(72) Inventor: LIAO, Yu-Hsuan, New Taipei City (TW); YEH, Tung-Lin, New Taipei City (TW); CHIEN, Huang-Chan, New Taipei City (TW)
(74) Representative: Viering, Jentschura & Partner mbB Patent- und Rechtsanwälte

(57) **Abstract**

An intraocular pressure sensing element (1, 1') and an intraocular pressure sensing method are provided. The intraocular pressure sensing element (1, 1') includes a lens (10), an annular strain gauge (12, 12'), regional strain gauges (14), and a sensing processing circuit (16). The lens (10) has a central region (A1) and a peripheral region (A2) surrounding the central region (A1). The annular strain gauge (12, 12') is disposed in the peripheral region (A2) and surrounds the central region (A1). The regional strain gauges (14) are arranged at predetermined positions in the peripheral region (A2), respectively. In response to the intraocular pressure sensing element (1, 1') being worn on a subject eyeball, the sensing processing circuit (16) measures the subject eyeball through the annular strain gauge (12, 12') to obtain first stress data, measures the subject eyeball through the regional strain gauges (14) to obtain second stress data corresponding to the plurality of predetermined positions, respectively, and generates intraocular pressure distribution information according to the first stress data and the second stress data.

## Description

### FIELD OF THE INVENTION

The present invention relates to a sensing element and a sensing method, and more particularly to a non-invasively intraocular pressure sensing element and a non-invasively intraocular pressure sensing method.

### BACKGROUND OF THE INVENTION

As people work longer hours spend more time using electronic products at close range, they are more likely to experience symptoms such as eye fatigue and excessive eye pressure due to overuse of the eyes, thereby accelerating eye aging and leading to severe myopia.

Generally speaking, high myopia, diabetes or hypertension patients, or those with a family medical history of glaucoma, are all at high risk of developing glaucoma, and in severe cases, can even lead to blindness. Therefore, timely monitoring of eye pressure is an extremely important part of maintaining eye health.

In the existing eye pressure measurement equipment, an embedded microelectronic system can be set up in disposable contact lenses to measure the change in corneal curvature caused by changes in eye pressure. However, in the current methodology for measuring eye pressure, due to irregular changes in the corneal curvature and cornea of each individual, corneoscleral junction angles are different, which leads to deviations in eye pressure measurements.

However, in order to accurately assess eye pressure in clinical practice, it is necessary to accurately obtain the pressure changes in each region of the cornea. Therefore, improving the eye pressure sensing element to overcome the above-mentioned problems has become one of the important issues to be solved in the relevant art.

### SUMMARY OF THE INVENTION

In response to the above-referenced technical inadequacies, the present invention provides a non-invasive intraocular pressure sensing element and a non-invasive intraocular pressure sensing method.

In order to solve the above-mentioned problems, one of the technical aspects adopted by the present invention is to provide an intraocular pressure sensing element, which includes a lens, an annular strain gauge, a plurality of regional strain gauges, and a sensing processing circuit. The lens has a central region and a peripheral region surrounding the central region. The annular strain gauge is disposed in the peripheral region and surrounds the central region. The plurality of regional strain gauges are arranged at a plurality of predetermined positions in the peripheral region, respectively. The sensing processing circuit is electrically connected to the annular strain gauge and the plurality of regional strain gauges. In response to the intraocular pressure sensing element being worn on a subject eyeball, the sensing processing circuit is configured to measure the subject eyeball through the annular strain gauge to obtain first stress data, to measure the subject eyeball through the plurality of regional strain gauges to obtain a plurality of records of second stress data corresponding to the plurality of predetermined positions, respectively, and to generate intraocular pressure distribution information according to the first stress data and the plurality of records of the second stress data.

In order to solve the above-mentioned problems, another one of the technical aspects adopted by the present invention is to provide an intraocular pressure sensing method, including: placing an intraocular pressure sensing element on a subject eyeball, in which the intraocular pressure sensing element includes a lens, an annular strain gauge, a plurality of regional strain gauges, and a sensing processing circuit. The lens has a central region and a peripheral region surrounding the central region. The annular strain gauge is disposed in the peripheral region and surrounds the central region. The plurality of regional strain gauges are arranged at a plurality of predetermined positions in the peripheral region, respectively. The sensing processing circuit is electrically connected to the annular strain gauge and the plurality of regional strain gauges. The intraocular pressure sensing method further includes configuring the sensing processing circuit to perform the following processes: measuring the subject eyeball through the annular strain gauge to obtain first stress data; measuring the subject eyeball through the plurality of regional strain gauges to obtain a plurality of records of second stress data corresponding to the plurality of predetermined positions, respectively; and generating intraocular pressure distribution information according to the first stress data and the plurality of records of the second stress data.

Therefore, in the intraocular pressure sensing element and intraocular pressure sensing method provided by the present invention, the annular strain gauge is used to measure a stress value of a main sensing region of the lens, and the plurality of regional strain gauges are arranged in an annular array at a plurality of predetermined positions around a subject eyeball, thereby reading stress value differences in the regions corresponding to the predetermined positions. The total stress value is compensated according to the stress value differences, thereby obtaining an accurate intraocular pressure change value when monitoring an actual intraocular pressure distribution of the subject eyeball.

In addition, the present invention also uses flexible capacitive pressure sensor (FCPS) to serve as the annular strain gauge, so as to measure the absolute pressure value of the main sensing region. Therefore, after multiple relative pressure difference values are obtained through the regional strain gauges, accurate intraocular pressure distribution information can be generated based on the absolute pressure value and the relative pressure difference values. In the FCPS, choosing a columnar structure for a flexible dielectric layer can maintain a large contact area between the dielectric layer and the electrode for enhancing sensitivity. The choice of using zinc oxide (ZnO) at a dielectric-electrode interface can also enhance a dielectric characteristic through Maxwell-Wagner-Sillars polarization effect, thereby improving the performance of the capacitive pressure sensor.

These and other aspects of the present invention will become apparent from the following description of the embodiment taken in conjunction with the following drawings and their captions, although variations and modifications therein may be affected without departing from the spirit and scope of the novel concepts of the invention.

### BRIEF DESCRIPTION OF THE DRAWINGS

The described embodiments may be better understood by reference to the following description and the accompanying drawings, in which:
FIG. 1 is a functional block diagram of an intraocular pressure sensing element according to a first embodiment of the present invention;
FIG. 2 is a schematic top view of the intraocular pressure sensing element according to the first embodiment of the present invention;
FIG. 3 is a flowchart of an intraocular pressure sensing method according to one embodiment of the present invention;
FIG. 4 is a schematic diagram of intraocular pressure distribution information according to one embodiment of the present invention;
FIG. 5 is a functional block diagram of a sensing processing circuit according to one embodiment of the present invention;
FIG. 6 is a schematic top view of an intraocular pressure sensing element according to another embodiment of the present invention; and
FIG. 7 is a schematic cross-sectional view along a section line A-A of FIG. 6.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

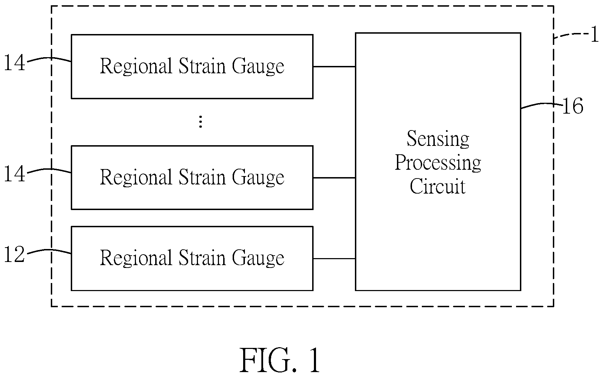

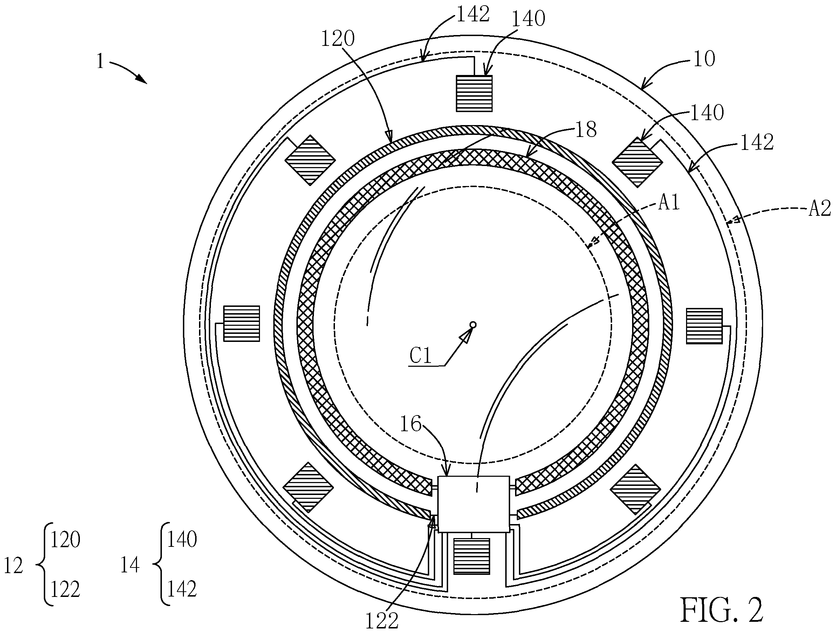

FIG. 1 is a functional block diagram of an intraocular pressure sensing element according to a first embodiment of the present invention, and FIG. 2 is a schematic top view of the intraocular pressure sensing element according to the first embodiment of the present invention. Referring to FIGS. 1 and 2, the first embodiment of the present invention provides an intraocular pressure sensing element 1, including a lens 10, an annular strain gauge 12, a plurality of regional strain gauges 14 and a sensing processing circuit 16.

In general, the lens 10 is a common contact lens, which can be made of a polymer (such as hydrogel or silicone hydrogel) with a specific shape for correction of various vision-related problems. The lens 10 has a central region A1 and a peripheral region A2 surrounding the central region A1. In this embodiment, the central region A1 can be, for example, a circular region extending outward from a central point C1 of the lens 10, and the peripheral region A2 can be, for example, an annular region with the central point C1 as the center and further extending outward from a circumference of the central region A1.

In this embodiment, the central region A1 corresponds to a visible part of the eyeball, such as the pupil, and the peripheral region A2 can correspond to the non-visible area of the eyeball, such as the iris, the cornea adjacent to the sclera, and the sclera. The central region A1 can be shaped to provide a function of correcting visual defects, and the so-called visual defects can include myopia (near-sightedness), hyperopia (far-sightedness), presbyopia and astigmatism. In some embodiments, the central region A1 can be provided without the function of correcting vision, but the present invention is not limited thereto.

The annular strain gauge 12 and the regional strain gauges 14 can be disposed on one surface of the lens 10 or embedded in the lens 10. The annular strain gauge 12 is disposed in the peripheral region A2 and is disposed around the central region A1. The regional strain gauges 14 are arranged at a plurality of predetermined positions in the peripheral region A2. The regional strain gauges 14 can be arranged in an annular array in the peripheral region A2. More specifically, the regional strain gauges 14 can be arranged equidistantly around the center point C1 of the lens 10 and outside the annular strain gauge 12. In this embodiment, a quantity of the regional strain gauges 14 can be eight, but the present invention is not limited thereto. Considering a configuration that enables the regional strain gauge 14 to perform basic functions, at least two regional strain gauges 14 are required, that is, the quantity of the regional strain gauges 14 must be greater than or equal to 2.

Furthermore, the sensing processing circuit 16 is electrically connected to the annular strain gauge 12 and the regional strain gauges 14 and can be, for example, an application specific integrated circuit (ASIC) chip that can perform signal processing, calculation, communication, and supply power.

Specifically, in the present invention, the annular strain gauge 12 is mainly used to measure a stress value of a main sensing region of the lens 10, and the plurality of regional strain gauges 14 are arranged in the annular array at the plurality of predetermined positions around the subject eyeball, thereby reading stress value differences in the regions respectively corresponding to the predetermined positions. The total stress value is compensated according to the stress value differences, thereby obtaining an accurate intraocular pressure change value when monitoring an actual intraocular pressure distribution of the subject eyeball.

In this embodiment, the annular strain gauge 12 and the regional strain gauges 14 can be, for example, resistive strain gauges that detect changes in resistances, so as to obtain stress. The annular strain gauge 12 includes an annular detection component 120 and a conductive wire 122, and each of the regional strain gauges 14 includes a regional detection component 140 and a conductive wire 142. The annular strain gauge 12 can be disposed inside the regional strain gauges 14, that is, a distance between the annular strain gauge 12 and the center point C1 is smaller than a distance between each of the regional strain gauges 14 and the center point C1.

In addition, a design with different strain coefficients for the annular strain gauge 12 and the regional strain gauges 14 are utilized in the present embodiment. For example, each of the regional strain gauge 14 has a first strain coefficient, the annular strain gauge 12 has a second strain coefficient, and the first strain coefficient is greater than the second strain coefficient. In detail, the higher the strain coefficient of the strain gauge, the more sensitive the strain gauge is to sensing stretching or deformation. Therefore, the annular strain gauge 12 can be made of a material with a low strain coefficient, and the regional strain gauge 14 can be made of a material with a high strain coefficient, so as to monitor eye pressure differences in different regions of the subject eyeball and reduce errors in the eye pressure detection caused by irregular changes in the human cornea. In some embodiments, the annular detection component 120 is made of an alloy material, such as nickel chromium or platinum, and the regional detection component 140 is made of a semiconductor material, such as graphite, carbon tube or transition metal such as tin selenide (SnSe2), molybdenum disulfide (MoS2) or tungsten diselenide (WSe2).

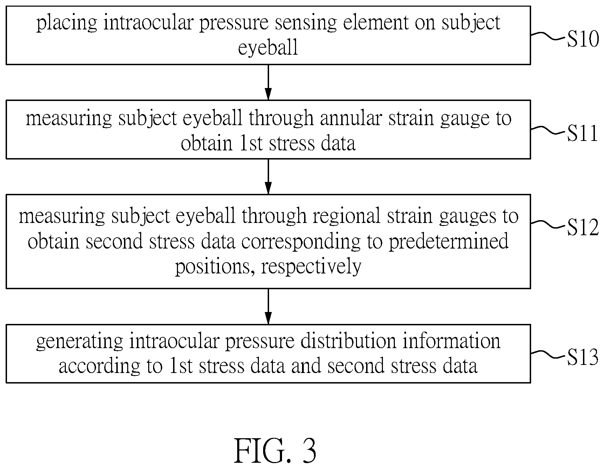

Reference is made to FIG. 3, which is a flowchart of an intraocular pressure sensing method according to one embodiment of the present invention. As shown in FIG. 3, the intraocular pressure sensing method of this embodiment includes the following processes:
Step S10: placing the intraocular pressure sensing element on a subject eyeball.

Next, the sensing processing circuit 16 is configured to perform the following processes.

Step S 11: measuring the subject eyeball through the annular strain gauge to obtain first stress data.

In step S 11, a first stress data can be obtained by detecting a resistance of the annular detection component 140, and a total resistance measured is associated with an intraocular pressure converted from a total area of the subject eyeball after deformation.

Step S12: measuring the subject eyeball through the plurality of regional strain gauges to obtain a plurality of records of second stress data corresponding to the plurality of predetermined positions, respectively. For example, each of the regional strain gauges 14 can measure the subject eyeball for a predetermined time period respectively at the corresponding predetermined positions, and observe stress differences generated in the predetermined time period to serve as the second stress data.

Step S13: generating intraocular pressure distribution information according to the first stress data and the plurality of records of the second stress data.

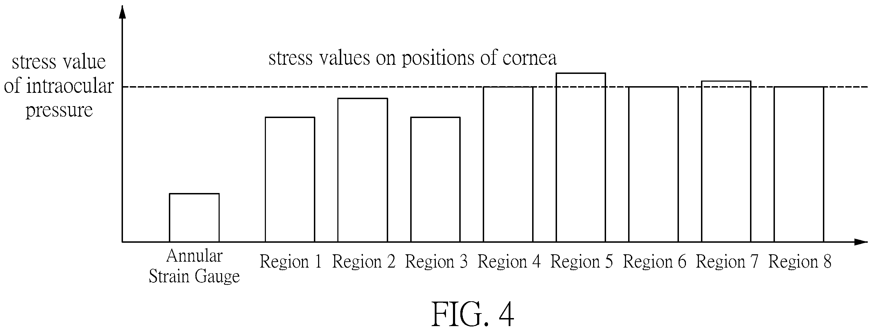

Referring to FIG. 4, FIG. 4 is a schematic diagram of intraocular pressure distribution information according to one embodiment of the present invention.

As shown in FIG. 4, after obtaining the total stress value measured by the annular strain gauge 14, a compensation can be performed according to the stress difference value obtained by each regional strain gauge 14 to obtain stress values of multiple regions, and the stress values can then be converted to obtain the intraocular pressure distribution. Therefore, regardless of the corneal curvature of each individual and how irregularly the cornea changes, the intraocular pressure sensing element of the present invention can accurately obtain the intraocular pressure changes in each region.

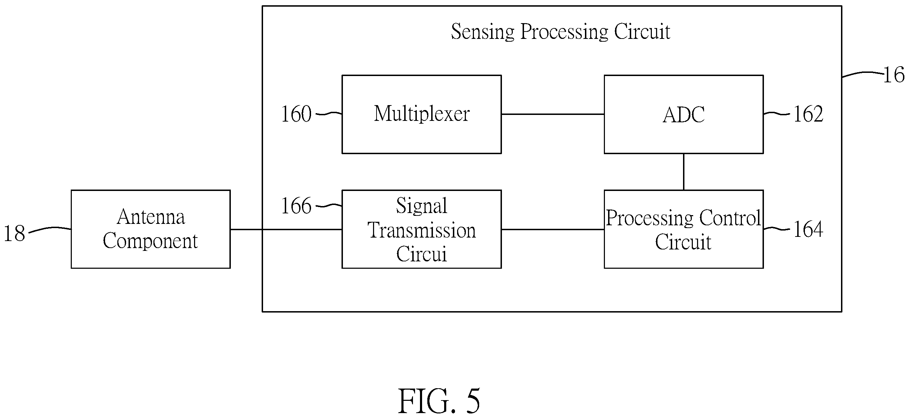

Furthermore, reference is made to FIG. 5, which is a functional block diagram of a sensing processing circuit according to one embodiment of the present invention. As shown in FIG. 5, the sensing processing circuit 16 can include a multiplexer 160, an analog-to-digital converter (ADC) 162, a processing control circuit 164, and a signal transmission circuit 166.

The multiplexer 160 can be electrically connected to the annular strain gauge 12 and the regional strain gauges 14, and can be controlled by the processing control circuit 164 to perform switching so as to select one or more of the annular strain gauge 12 and the regional strain gauges 14 as stress value input source(s). The analog-to-digital converter 162 is configured to convert signals from the annular strain gauge 12 and the regional strain gauges 14. For example, the analog-to-digital converter 162 can transmit voltage signals generated by the annular strain gauge 12 and the regional strain gauges 14 to the processing control circuit 164 in a form of digital signals.

The processing control circuit 164 can be, for example, a processor for processing the converted signals (digital signals) of the annular strain gauge 12 and the regional strain gauges 14 to obtain the first stress data and the second stress data, and performing processing to generate intraocular pressure distribution data.

Referring to FIG. 1 again, the intraocular pressure sensing element 1 further includes an antenna component 18, and the antenna component 18 has a ring shape and is arranged around the central region A 1. The antenna component 18 is electrically connected to the sensing processing circuit 16 and is disposed in the peripheral region A2 for data transmission with an external device. In this embodiment, in order to avoid interference, the annular strain gauge 12, the regional strain gauges 14 and the antenna component 18 do not overlap each other in a normal direction of the lens 10.

The signal transmission circuit 166 of the sensing processing circuit 16 can be electrically connected to the antenna component 18, and is configured to receive data or transmit the intraocular pressure distribution data through the antenna component 18. The signal transmission circuit 166 and the antenna component 18 can also communicate with various electronic devices for various applications. For example, in other embodiments, the intraocular pressure sensing element 1 can be wirelessly connected to any wearable device worn by a user (e.g., a reader mounted on glasses or a neck-mounted reader), and the above-mentioned wearable devices (or readers) can adopt common wireless transmission technology (e.g., RFID) or other wireless electrically-inductive technology to supply power, perform sensing, or feedback signals to the intraocular pressure sensing element 1.

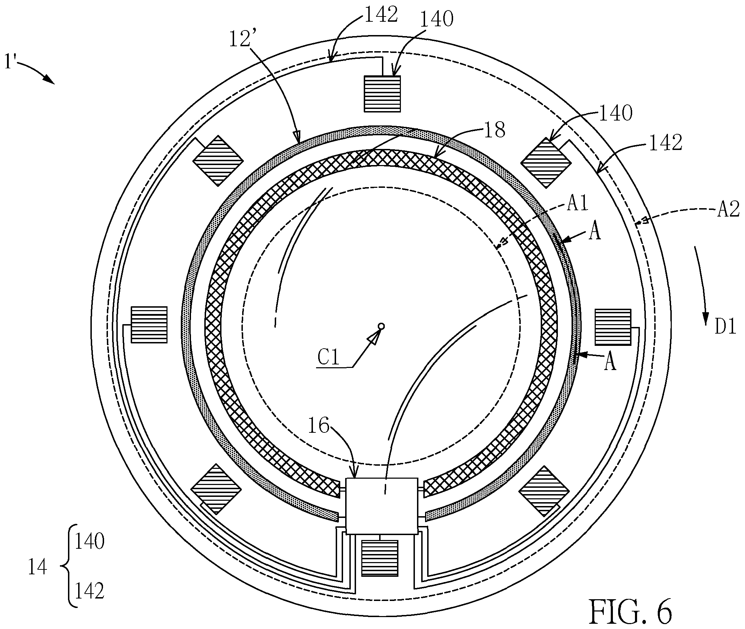

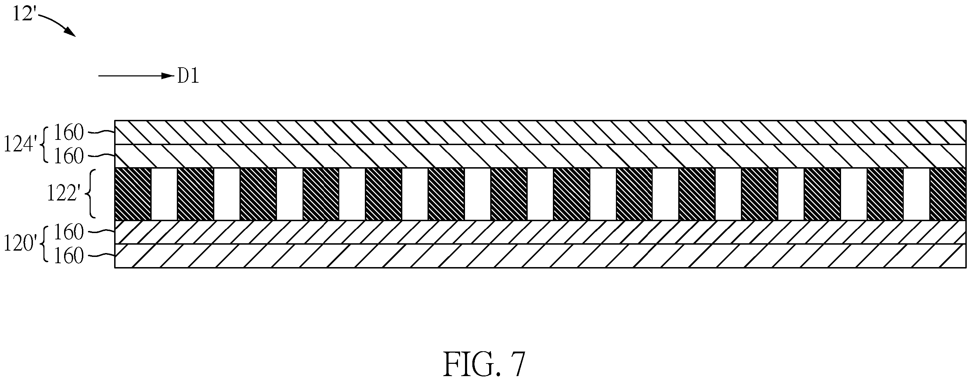

FIG. 6 is a schematic top view of an intraocular pressure sensing element according to another embodiment of the present invention, and FIG. 7 is a schematic cross-sectional view along a section line A-A of FIG. 6. In this embodiment, the annular strain gauge 12' is a flexible capacitive pressure sensor (FCPS), which includes a bottom electrode layer 120', a flexible dielectric layer 122', and a top electrode layer 124'. The flexible dielectric layer 122' is disposed on the bottom electrode layer 120', and the top electrode layer 124' is disposed on the flexible dielectric layer 122'.

In the capacitive pressure sensor used in this embodiment, the flexible dielectric layer 122' is placed between the top electrode layer 124' and the bottom electrode layer 120'. When an external force is applied to the sensor, the flexible dielectric layer 122' undergoes elastic deformation, which causes a distance between electrodes to decrease, thereby increasing a total capacitance of the sensor.

In addition, the flexible dielectric layer 122' includes a plurality of elastic columns FC which are spaced apart from one another along a circumferential direction D1 of the peripheral region A2. A height of the elastic columns FC preferably ranges from 0.1 µm to 2 µm, and a width of the elastic column FC preferably ranges from 100 nm to 1000 nm. The elastic columns FC can be made of polydimethylsiloxane polymer (PDMS). A columnar structure of the flexible dielectric layer 122' can provide large contact areas between the dielectric layer and the electrode, and the FCPS formed with the columnar structure has high sensitivity in both an ultra-low pressure range and a high pressure range. The ultra-low pressure range preferably ranges from 130 Pa to 6700 Pa, and the high pressure range preferably ranges from 67000 Pa to 102000 Pa.

On the other hand, the bottom electrode layer 120' and the top electrode layer 124' can each be provided with a stacked structure. For example, the bottom electrode layer 120' can include a first transparent conductive layer E1 and a second transparent conductive layer E2 disposed on the first transparent conductive layer E1, and the top electrode layer 124' can include a third transparent conductive layer E3 and a fourth transparent conductive layer E4 disposed on the third transparent conductive layer E3. The second transparent conductive layer E2 and the third transparent conductive layer E3 can be made of zinc oxide (ZnO), and the first transparent conductive layer E1 and the fourth transparent conductive layer E4 can be made of indium tin oxide (ITO). It should be noted that since zinc oxide is easily polarized by an external electric field, a dielectric characteristic can be enhanced through Maxwell-Wagner-Sillars polarization effect, thereby improving the performance of the capacitive pressure sensor. In addition, when the second transparent conductive layer E2 and the third transparent conductive layer E3 are made of zinc oxide (ZnO), high transmittance can be achieved, which makes the thin film structure transparent.

Based on the above structure, when the FCPS is used as the annular strain gauge 12', the first stress data measured is an absolute pressure value. In addition, when the intraocular pressure sensing element 1' is worn on the subject eyeball, the intraocular pressure sensing element 1' will bend according to a curvature of the cornea. When the intraocular pressure increases, a radius of curvature of the intraocular pressure sensing element 1' increases accordingly, and a degree of curvature also changes accordingly, such that the FCPS possesses a change in the capacitance as the intraocular pressure of the subject eyeball changes, thereby enabling the change of intraocular pressure to be monitored in real time. Therefore, after obtaining multiple relative pressure differences through the regional strain gauges 14, the sensing processing circuit 16 can compensate the absolute pressure value of the main sensing region according to the relative pressure differences to generate accurate intraocular pressure distribution information.

### [Beneficial Effects of the Embodiments]

In conclusion, in the intraocular pressure sensing element and intraocular pressure sensing method provided by the present invention, the annular strain gauge is used to measure a stress value of a main sensing region of the lens, and the plurality of regional strain gauges are arranged in an annular array at a plurality of predetermined positions around the subject eyeball, thereby reading stress value differences in the regions corresponding to the predetermined positions. The total stress value is compensated according to the stress value differences, thereby obtaining an accurate intraocular pressure change value when monitoring an actual intraocular pressure distribution of the subject eyeball.

In addition, the present invention also uses FCPS to serve as the annular strain gauge, so as to measure the absolute pressure value of the main sensing region. Therefore, after multiple relative pressure difference values are obtained through the regional strain gauges, accurate intraocular pressure distribution information can be generated based on the absolute pressure value and the relative pressure difference values. In the FCPS, choosing a columnar structure for a flexible dielectric layer can maintain a large contact area between the dielectric layer and the electrode for enhancing sensitivity. The choice of using zinc oxide (ZnO) at a dielectric-electrode interface can also enhance a dielectric characteristic through Maxwell-Wagner-Sillars polarization effect, thereby improving the performance of the capacitive pressure sensor.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its spirit and scope.

## Claims

1. An intraocular pressure sensing element (1, 1'), **characterized by** comprising:
a lens (10) having a central region (A1) and a peripheral region (A2) surrounding the central region (A1);
an annular strain gauge (12) disposed in the peripheral region (A2) and surrounding the central region (A1);
a plurality of regional strain gauges (14) arranged at a plurality of predetermined positions in the peripheral region (A2), respectively; and
a sensing processing circuit (16) electrically connected to the annular strain gauge (12) and the plurality of regional strain gauges (14),
wherein, in response to the intraocular pressure sensing element (1, 1') being worn on a subject eyeball, the sensing processing circuit (16) is configured to measure the subject eyeball through the annular strain gauge (12) to obtain first stress data, to measure the subject eyeball through the plurality of regional strain gauges (14) to obtain a plurality of records of second stress data corresponding to the plurality of predetermined positions, respectively, and to generate intraocular pressure distribution information according to the first stress data and the plurality of records of the second stress data.

2. The intraocular pressure sensing element (1, 1') according to claim 1, wherein the central region (A1) is circular, the peripheral region (A2) is annular, the plurality of regional strain gauges (14) are arranged in an annular array in the peripheral region (A2), and a quantity of the plurality of regional strain gauges (14) is greater than or equal to 2.

3. The intraocular pressure sensing element (1, 1') according to claim 2, wherein the plurality of regional strain gauges (14) are equidistantly arranged around a center of the lens (10) and outside the annular strain gauge (12).

4. The intraocular pressure sensing element (1, 1') according to claim 1, wherein each of the plurality of regional strain gauges (14) has a first strain coefficient, the annular strain gauge (12, 12') has a second strain coefficient, and the first strain coefficient is greater than the second strain coefficient.

5. The intraocular pressure sensing element (1, 1') according to claim 4, wherein the annular strain gauge (12, 12') includes an annular detection component (120), and each of the plurality of regional strain gauges (14) includes a regional detection component (140), the annular detection component (120) is made of an alloy material, and the regional detection component (140) is made of a semiconductor material.

6. The intraocular pressure sensing element (1, 1') according to claim 1, wherein the annular strain gauge (12, 12') is a flexible capacitive pressure sensor, including:
a lower electrode layer (120');
a flexible dielectric layer (122') disposed on the lower electrode layer (120'); and
an upper electrode layer (124') disposed on the flexible dielectric layer (122').

7. The intraocular pressure sensing element (1, 1') according to claim 6, wherein the peripheral region (A2) is annular, and the flexible dielectric layer (122') includes a plurality of elastic columns (EC) spaced apart from one another along a circumferential direction of the peripheral region (A2).

8. The intraocular pressure sensing element (1, 1') according to claim 7, wherein the lower electrode layer (120') includes:
a first transparent conductive layer (E1); and
a second transparent conductive layer (E2) disposed on the first transparent conductive layer (E1);
wherein the upper electrode layer (124') includes:
a third transparent conductive layer (E3); and
a fourth transparent conductive layer (E4) disposed on the third transparent conductive layer (E3),
wherein the second transparent conductive layer (E2) and the third transparent conductive layer (E3) include zinc oxide, the first transparent conductive layer (E1) and the fourth transparent conductive layer (E4) include indium tin oxide, and the elastic columns (EC) each include polydimethylsiloxane polymer (PDMS).

9. The intraocular pressure sensing element (1, 1') according to claim 6, wherein the first stress data includes an absolute pressure value, the plurality of records of the second stress data include a plurality of relative pressure difference values, and the sensing processing circuit (16) is further configured to generate the intraocular pressure distribution information according to the absolute pressure value and the relative pressure difference values.

10. The intraocular pressure sensing element (1, 1') according to claim 1, further comprising an antenna component (18) electrically connected to the sensing processing circuit (16), wherein the antenna component (18) is disposed in the peripheral region (A2) for data transmission with an external device
wherein the sensing processing circuit (16) includes:
a multiplexer (160) electrically connected to the annular strain gauge (12, 12') and the plurality of regional strain gauges (14);
an analog-to-digital converter (162) configured to perform signal conversion on signals from the annular strain gauge (12, 12') and the plurality of regional strain gauges (14);
a processing control circuit (164) configured to process the converted signals of the annular strain gauge (12, 12') and the plurality of regional strain gauges (14) to obtain the first stress data and the plurality of records of the second stress data, wherein the processing control circuit (164) is further configured to process the first stress data and the plurality of records of the second stress data to generate the intraocular pressure distribution information; and
a signal transmission circuit (166) configured to receive data or transmit the intraocular pressure distribution data through the antenna component (18).

11. The intraocular pressure sensing element (1, 1') according to claim 10, wherein the antenna component (18) has a ring shape and is arranged around the central region (A1), and the annular strain gauge (12, 12'), the plurality of regional strain gauges (14) and the antenna component (18) do not overlap with one another in a normal direction of the lens (10).

12. An intraocular pressure sensing method, **characterized by** comprising:
placing an intraocular pressure sensing element (1, 1') on a subject eyeball, wherein the intraocular pressure sensing element (1, 1') includes:
a lens (10) having a central region (A1) and a peripheral region (A2) surrounding the central region (A1);
an annular strain gauge (12, 12') disposed in the peripheral region (A2) and surrounding the central region (A1);
a plurality of regional strain gauges (14) arranged at a plurality of predetermined positions in the peripheral region (A2), respectively; and
a sensing processing circuit (16) electrically connected to the annular strain gauge (12, 12') and the plurality of regional strain gauges (14); and
configuring the sensing processing circuit (16) to perform the following processes:
measuring the subject eyeball through the annular strain gauge (12, 12') to obtain first stress data;
measuring the subject eyeball through the plurality of regional strain gauges (14) to obtain a plurality of records of second stress data corresponding to the plurality of predetermined positions, respectively; and
generating intraocular pressure distribution information according to the first stress data and the plurality of records of the second stress data.

13. The intraocular pressure sensing method according to claim 12, wherein the central region (A1) is circular, the peripheral region (A2) is annular, the plurality of regional strain gauges (14) are arranged in an annular array in the peripheral region (A2), and a quantity of the plurality of regional strain gauges (14) is greater than or equal to 2.

14. The intraocular pressure sensing method according to claim 12, wherein each of the plurality of regional strain gauges (14) has a first strain coefficient, the annular strain gauge (12, 12') has a second strain coefficient, and the first strain coefficient is greater than the second strain coefficient; and
wherein the annular strain gauge (12, 12') includes an annular detection component (120), and each of the plurality of regional strain gauges (14) includes a regional detection component (140), the annular detection component (120) is made of an alloy material, and the regional detection component (140) is made of a semiconductor material.

15. The intraocular pressure sensing method according to claim 12, wherein the annular strain gauge (12, 12') is a flexible capacitive pressure sensor, including:
a lower electrode layer (120');
a flexible dielectric layer (122') disposed on the lower electrode layer (120'); and
an upper electrode layer (124') disposed on the flexible dielectric layer (122'),
wherein the peripheral region (A2) is annular, and the flexible dielectric layer (122') includes a plurality of elastic columns (EC) spaced apart from one another along a circumferential direction of the peripheral region (A2);
wherein the second transparent conductive layer (E2) and the third transparent conductive layer (E3) include zinc oxide, the first transparent conductive layer (E1) and the fourth transparent conductive layer (E4) include indium tin oxide, and the elastic columns (EC) each include polydimethylsiloxane polymer (PDMS),
wherein the first stress data includes an absolute pressure value, the plurality of records of the second stress data include a plurality of relative pressure difference values, and the sensing processing circuit (16) is further configured to generate the intraocular pressure distribution information according to the absolute pressure value and the relative pressure difference values.
